# EUROPEAN PATENT SPECIFICATION

(11) **EP 4 497 460 B1**
(45) Date of publication and mention of the grant of the patent: **23.09.2026**
(21) Application number: 23774306.7
(22) Date of filing: 14.02.2023
(51) Int. Cl.: A61M 16/00, A61M 16/06, A61B 5/08, A61B 5/113, A61B 5/389

(54) **RESPIRATORY SUPPORT SYSTEM**
ATEMUNTERSTÜTZUNGSSYSTEM
SYSTÈME D'ASSISTANCE RESPIRATOIRE

(30) Priority: 23.03.2022 JP 2022046788; 12.04.2022 JP 2022065936; 06.09.2022 JP 2022141401
(43) Date of publication of application: 29.01.2025
(73) Proprietor: CYBERDYNE INC., Tsukuba-shi, Ibaraki 305-0818 (JP)
(72) Inventor: SANKAI, Yoshiyuki, Tsukuba-shi, Ibaraki 305-0818 (JP)
(74) Representative: Mewburn Ellis LLP
(86) International application number: PCT/JP2023/004904
(87) International publication number: WO 2023/181707

(56) References cited:
- WO-A1-2010/115166
- WO-A1-2014/162283
- WO-A1-99/13931
- WO-A2-2007/123899
- JP-A- 2012 522 608
- JP-A- 2013 517 827
- JP-A- 2016 517 726
- JP-B1- S4 925 036

## Description

### Technical Field

The present invention relates to a portable respiratory assistance system that noninvasively assists respiratory of a subject.

### Background Art

Progressive neuromuscular diseases such as congenital myopathy, amyotrophic lateral sclerosis (ALS) and muscular dystrophy cause muscle atrophy and/or muscle weakness and result in disorders in functions of four limbs, speech, swallowing, respiratory functions and/or the like. As weakening of respiratory muscle and airway muscle including the diaphragm progresses, a respiratory function disorder such as difficult breathing during activities of daily living and lack of breathing or hypopnoea is caused. Those may cause a symptom in daily life such as fatigue, headache, and drowsiness, which significantly lowers patient's quality of life (QOL) or activities of daily living (ADL).

While a respiratory functional disorder of some patients suffering a neuromuscular disease is often considered as a terminal symptom, cases in which such patients maintaining functions such as walking, talking and swallowing although weakening of respiratory muscles precedes thereto have been reported. Assisting the respiratory function of a subject suffering a respiratory functional disorder by means of an artificial respirator allows the subject to keep his or her daily life.

As management for such an artificial respirator, it has been a common practice to perform noninvasive positive-pressure ventilation (NPPV). An artificial respiration system applying the NPPV eliminates the need for tracheal intubation using a mask and generally employs noninvasive artificial respirator that assists inhalation and exhalation (see Patent Literature 1, for example).

Since the NPPV can be easily introduced, interrupted, and withdrawn in noninvasive manner and allows talking and eating, it is a preferable option for a subject who has a good conscious level and can perform spontaneous respiration.

For use as a portable apparatus, the artificial respirator is received in a rack capable of being wheeled so that it is movable integrally with the rack within a hospital. Since an artificial respirator must be provided with many functions to support various medical conditions and severities of illnesses, many artificial respirator products are relatively large in size even without their rack and weigh 5 to 10 [kg].

### Citation List

### Patent Literature

Patent Literature 1: Japanese Patent Laid-Open No. 2020-535909

WO2010115166A1 discloses a non-invasive open ventilation system using one or more free-space nasal jet nozzles aligned with the nostrils to deliver pressurised ventilation gas that entrains ambient air while allowing spontaneous breathing. The system includes support/positioning structures, breath sensing near the nose, and ventilator control synchronised with breathing phases, optionally for respiratory support or sleep-apnoea treatment.

### Summary of Invention

### Technical Problem

By the way, in a case where a subject is suffering an intractable neurological or muscle disease, since the subject comes to have difficulty in spontaneous respiration with progression of the intractable disease, it is desirable to try performing locomotion such as actively walking outside while the subject has muscular strength for moving autonomously.

However, a subject using a relatively heavy artificial respirator as in Patent Literature 1 and has a respiratory function which is depressed before functions of the four limbs is forced to stay in bed or to live on an electric wheel chair though the subject has muscular strength allowing self-walking, possibly forming a vicious spiral of reduced self-support degrees and disuses of his or her muscular strength.

Furthermore, a general artificial respirator is provided with a function for forcibly performing ventilation at predetermined intervals regardless of the intention to breath of the subject, and ventilation may be performed in a state that spontaneous respiration which is called asynchronous timing ventilation and respiration assistance are mistimed. Ventilation using such an unsuitably-timed artificial respirator hurts or discomforts the subject, and relevance to problems leading an increased number of years of use of the artificial respirator has been pointed out.

Accordingly, in a case where the subject has a sufficient walking function, the subject desirably wears on his or her body and carries an artificial respirator which can provide respiration assistance as needed so that the subject can perform outdoor walking, allowing maintenance of his or her motor function and prevention of reduction of the subject's QOL and ADV.

The present invention has been made in consideration of those points above and aims to propose a respiratory assistance system which can assist independent life of the subject by keeping the subject's motor functions as much as possible.

### Solution to Problem

In order to solve the problem, the present invention is configured to include a portable artificial respirator that noninvasively assists respiratory of a subject, a respiratory circuit having one end to which the artificial respirator is connected and another end to which a mouthpiece is connected, a neck holder configured such that the mouthpiece is placed in vicinity of the mouth of the subject when the neck holder is attached to the neck of the subject from his or her back, and a contact sensor that is provided at a tip of the mouthpiece and that detects a contact state when the subject holds the mouthpiece in his or her mouth, wherein the artificial respirator controls a start or end of respiratory assistance to the subject based on a detection result of the contact sensor.

As a result, in the respiratory assistance system, the subject can immediately receive respiratory assistance by the artificial respirator only by holding the mouthpiece in his or her mouth as needed while walking. The wearable configuration with the combination of the neck holder and the mouthpiece allows the subject to receive respiratory assistance only as needed without covering the whole face of the subject like a mask.

According to the present invention, a signal detecting unit is further provided that is removably attached to a lower part of the chest of the subject and that detects a bioelectrical signal generated by shrinking and relaxing of the diaphragm with spontaneous respiration of the subject, and the artificial respirator controls either one or both of the ventilation amount and air pressure of the pressurized air to be supplied to the respiratory circuit so as to be synchronized with inhalation or exhalation timing of the subject based on a detection result from the signal detecting unit.

As a result, in the respiratory assistance system, when the subject holds the mouthpiece in his or her mouth, the respiratory circuit can be supplied with pressurized air in synchronization with abdominal breathing timing of the subject during respiratory assistance to the subject.

According to the present invention, a vibration detecting unit is further included that is removably attached to a part near the chest of the subject and that detects a lung activity with spontaneous respiration of the subject, and the artificial respirator controls either one or both of the ventilation amount and air pressure of the pressurized air to be supplied to the respiratory circuit so as to be synchronized with inhalation or exhalation timing of the subject based on a detection result from the vibration detecting unit.

As a result, in the respiratory assistance system, when the subject holds the mouthpiece in his or her mouth, the respiratory circuit can be supplied with pressurized air in synchronization with timing of slight movements of the lung with spontaneous respiration of the subject during respiratory assistance to the subject.

According to the present invention, a signal detecting unit that is removably attached to a lower part of the chest of the subject and that detects a bioelectrical signal generated by shrinking and relaxing of the diaphragm with spontaneous respiration of the subject and a vibration detecting unit that is removably attached to a part near the chest of the subject and that detects a lung activity with spontaneous respiration of the subject are further provided, and the artificial respirator controls either one or both of the ventilation amount and air pressure of the pressurized air to be supplied to the respiratory circuit so as to be synchronized with inhalation or exhalation timing of the subject based on a detection result from the signal detecting unit and the vibration detecting unit.

As a result, in the respiratory assistance system, when the subject holds the mouthpiece in his or her mouth, the respiratory circuit can be supplied with pressurized air in synchronization with abdominal breathing timing of the subject during respiratory assistance to the subject and, at the same time, the respiratory circuit can be supplied with pressurized air to the artificial respirator in synchronization with timing of slight movements of the lung with spontaneous respiration of the subject during respiratory assistance to the subject.

Furthermore, in the present invention, the artificial respirator is configured to have a wearable size and weight so that the artificial respirator can be attached to the body of the subject and is held on the body of the subject via a holder. As a result, with the respiratory assistance system, the subject can carry the artificial respirator held via the holder on his or her body without imposing any problem on daily life.

Furthermore, according to the present invention, the artificial respirator is configured to have a battery and an operation interface arranged in parallel on an exposed surface of the subject to which the artificial respirator is attached, the operation interface including a display function. As a result, with the respiratory assistance system, the subject when going out can easily perform an operation necessary for respiratory assistance and battery replacement by keeping the artificial respirator to be carried with the subject.

### Advantageous Effects of Invention

According to the present invention, a respiratory assistance system can be implemented which can assist independent life of the subject by keeping the subject's motor functions as much as possible.

### Brief Description of Drawings

[Figure 1] Figure 1 is an external perspective view showing a configuration of a respiratory assistance system according to a first embodiment.
[Figure 2] Figure 2 is a schematic diagram for explanation of a state that the respiratory assistance system shown in Figure 1 is attached to a subject.
[Figure 3] Figure 3 is a block diagram showing an internal configuration of an artificial respirator according to the first embodiment.
[Figure 4] Figure 4 is a block diagram regarding pressure control.
[Figure 5] Figure 5 is a schematic diagram showing a usage state when a subject receives respiratory assistance.
[Figure 6] Figure 6 is a schematic diagram for explanation of experiment steps.
[Figure 7] Figure 7 is a graph showing an experiment result by a mouthpiece holding action detection algorithm.
[Figure 8] Figure 8 is a table showing experiment results by the mouthpiece holding action detection algorithm.
[Figure 9] Figure 9 is an external perspective view showing a configuration of a respiratory assistance system according to a second embodiment.
[Figure 10] Figure 10 is a block diagram showing an internal configuration of an artificial respirator according to the second embodiment.
[Figure 11] Figure 11 is a schematic diagram showing a positional relationship among breast bones, the diaphragm, external intercostal muscles and positions of electrodes when measuring bioelectrical signals.
[Figure 12] Figure 12 is a graph showing signal processing flows for spontaneous respiration detection and respiratory assistance using bioelectrical signals of a respiratory muscle.
[Figure 13] Figure 13 is a graph showing an experiment result by a spontaneous respiration detection algorithm using bioelectrical signals of a respiratory muscle.
[Figure 14] Figure 14 is a table showing experiment results by the spontaneous respiration detection algorithm using bioelectrical signals of a respiratory muscle.

### Description of Embodiments

Embodiments of the invention are described in detail below with reference to drawings.

### (1) Configuration of Respiratory Assistance System According to First Embodiment

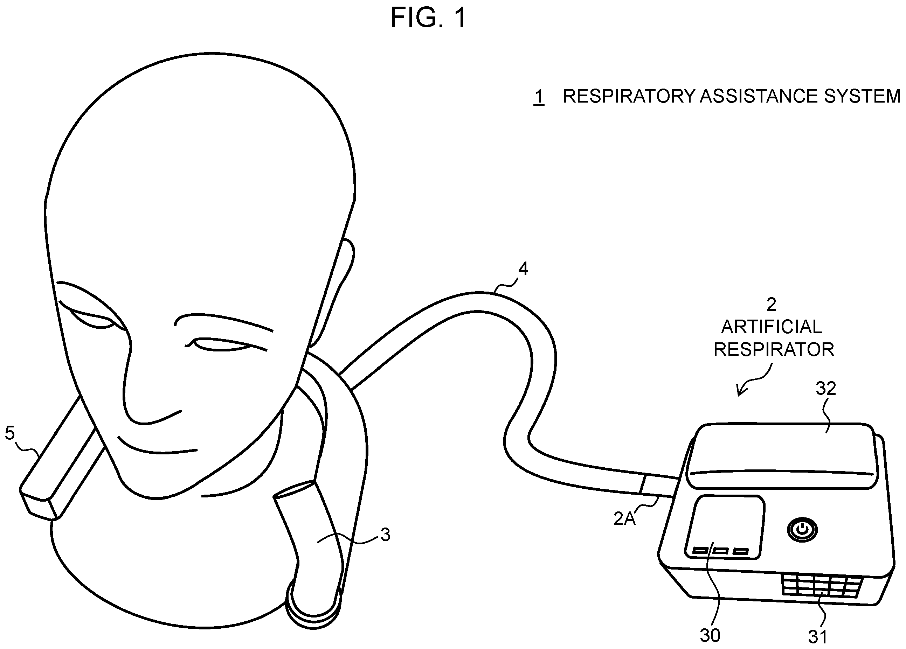

Figure 1 is an external perspective view showing a respiratory assistance system 1 according to a first embodiment. The respiratory assistance system 1 has a portable artificial respirator 2 that noninvasively assist respiratory of a subject, a respiratory circuit 4 that connects the artificial respirator 2 and a mouthpiece 3, and a neck holder 5 that holds the mouthpiece 3.

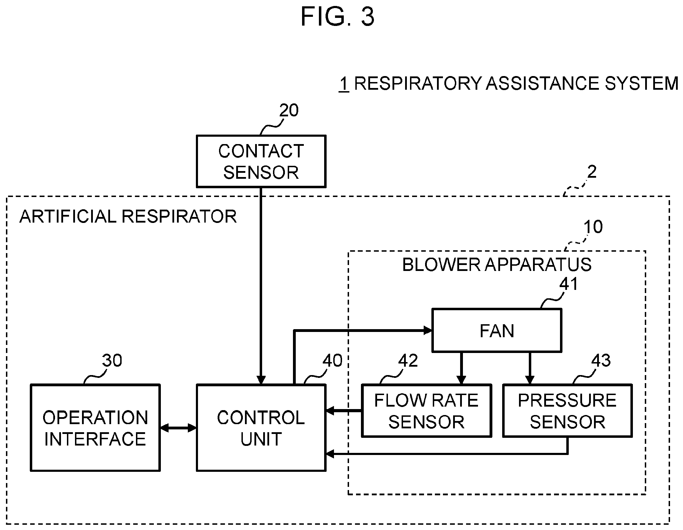

The artificial respirator 2 is a positive-pressure artificial respirator internally contains a blower apparatus 10 (see Figure 3, which will be described later) and supplies air with controlled pressure and volume to a subject through the respiratory circuit 4 based on settings for a desired ventilation condition. This artificial respirator 2 has wearable sizes (width 158[mm], height 68[mm], depth 128[mm]) and weight (about 1[kg]) allowing attachment to the body of a subject.

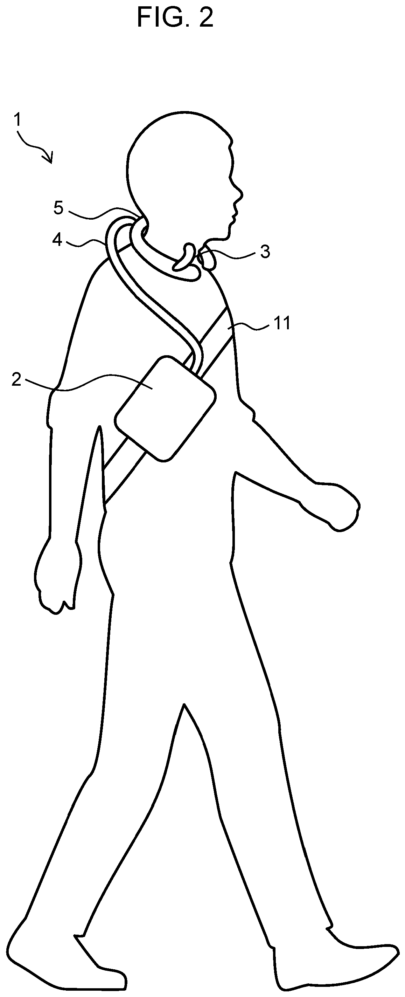

Therefore, this artificial respirator 2 can be held by the body of a subject via a shoulder belt (holder) 11 shown in Figure 2, and the subject can carry the artificial respirator 2 held on the body via the shoulder belt 11 with no problem on daily life.

Referring to Figure 1, the respiratory circuit 4 includes a plurality of hoses (tube for air sending), a Y piece, a water trap, an exhalation valve and so on (not all shown), and has one end having an intake port 2A to which the artificial respirator 2 is connected and another end to which the mouthpiece 3 is connected.

The neck holder 5 is configured to have a substantial U-shape such that the mouthpiece 3 is placed in vicinity of the mouth of the subject when the neck holder 5 is attached to the neck of the subject from the back. The mouthpiece 3 has an end having a contact sensor 20 (see Figure 3 which will be described later) thereon and is configured to detect a contact state when the subject holds the mouthpiece 3 in his or her mouth.

The contact sensor 20 has an electrostatic capacity switch that can sense a contact of a human body, is placed at a circumference of the end of the mouthpiece 3 and detects a contact state when the subject holds the mouthpiece 3 in his or her mouth upon contact of the lips of the subject.

The artificial respirator 2 has an operation interface 30 including a display function on one surface (exposed surface when it is attached to the subject). Furthermore, an air filter 31 serving as an air intake port from outside on a side of the body part of the artificial respirator 2.

On one surface of the body part of the artificial respirator 2, a removable portable battery 32 is also placed in parallel with the operation interface 30. The artificial respirator 2 has a power supply terminal with which an AC adapter (not shown) is connectable to an external power supply and is configured to allow charging portable battery 32 attached to the body part.

Figure 3 is a block diagram showing a configuration of a control-related system of the artificial respirator 2 in the respiratory assistance system 1. The artificial respirator 2 has a control unit 40 including a central processing unit (CPU) that generally control the whole system and a blower apparatus 10.

The blower apparatus 10 has a fan 41 having a centrifugal micro blower configuration including an actuator and an impeller, a flow rate sensor 42 having an ultrasonic flowmeter, and a diaphragm type pressure sensor 43 using MEMS technology and generates pressurized air by compressing the air externally taken via an air filter 31 under control of the control unit 40.

When the subject sets various ventilation conditions such as an air supplying time and assistance pressure as desired conditions by using the operation interface 30, the control unit 40 can control the set pressure, capacity, flow rate and so on with reference to the detection results from the flow rate sensor 42 within the blower apparatus 10 and the pressure sensor 43 to supply pressurized air to the respiratory circuit 4.

In the artificial respirator 2, the control unit 40 controls a start or end of respiratory assistance to the subject based on a detection result of the contact sensor 20 provided on the mouthpiece 3. In other words, if the contact sensor 20 detects that the subject holds the mouthpiece 3 in his or her mouth, the control unit 40 assists respiratory by supplying pressurized air from the artificial respirator 2 through the respiratory circuit 4 to the subject and, if the fact that the subject has detached the mouthpiece 3 is detected, stops the control and stops the respiratory assistance.

Note that it configured such that the auxiliary oxygen supply (not shown) is connectable to the artificial respirator 2, and a subject may control the pressure, capacity, a flow rate and so on to be set by using the operation interface 30 as needed to regulate the oxygen concentration of mixed gas of oxygen supplied from the oxygen supply and air and supply the mixed gas to the respiratory circuit 4.

For providing respiratory assistance to a subject, the control unit 40 sets the relationship between an inhalation time and an exhalation time (I:E ratio) to 1:3, and the subject sets the inhalation time while the exhalation time is determined in accordance with the I:E ratio. It should be noted that new respiratory assistance is not performed during exhalation.

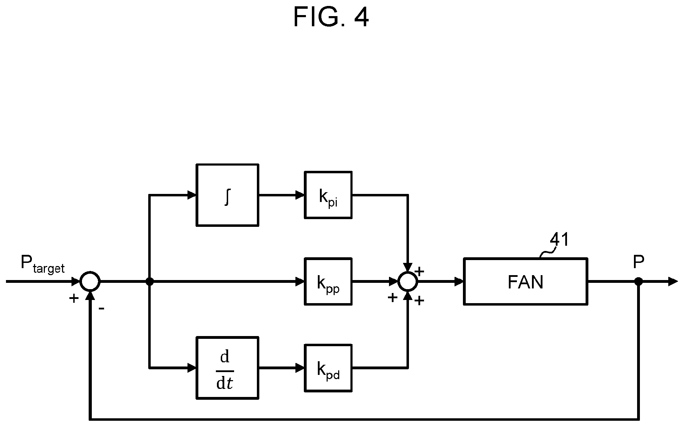

Now, a respiration assistance pressure control algorithm by the control unit 40 is described. In order to perform pressure control, the control unit 40 feeds back a differential value and an integrated value for a difference between a target pressure value P_{target} and a pressure value P measured by the pressure sensor 43 and applies PID control to the motor rotation speed ω of the fan 41 to provide assistance with the target pressure. A block diagram regarding actual pressure control is shown in Figure 4. Referring to Figure 4, kₚₚ, kₚᵢ, k_{pd} indicate respective gains of the PID control.

Next, a detection algorithm for mouthpiece holding action by the control unit 40 is described. In a standby state where the artificial respirator 2 is not assisting respiration, the mouthpiece 3 is separated from the mouth of the subject. In this state, the flow rate is higher than that when the subject is holding the mouthpiece 3 in his or her mouth.

Therefore, if the flowrate within the respiratory circuit 4 is less than a threshold, the control unit 40 determines that the subject has requested respiratory assistance and has held the mouthpiece 3 in his or her mouth and performs respiratory assistance. The artificial respirator 2 is set to the standby state after the respiratory assistance by the control unit 40. In the standby state, the mouth of the subject is separated from the mouthpiece 3, and the flow rate within the respiratory circuit 4 is constant at a high level. On the other hand, if the measured flow rate within the respiratory circuit 4 exceeds the predetermined threshold, the control unit 40 determines that the standby state is acquired again.

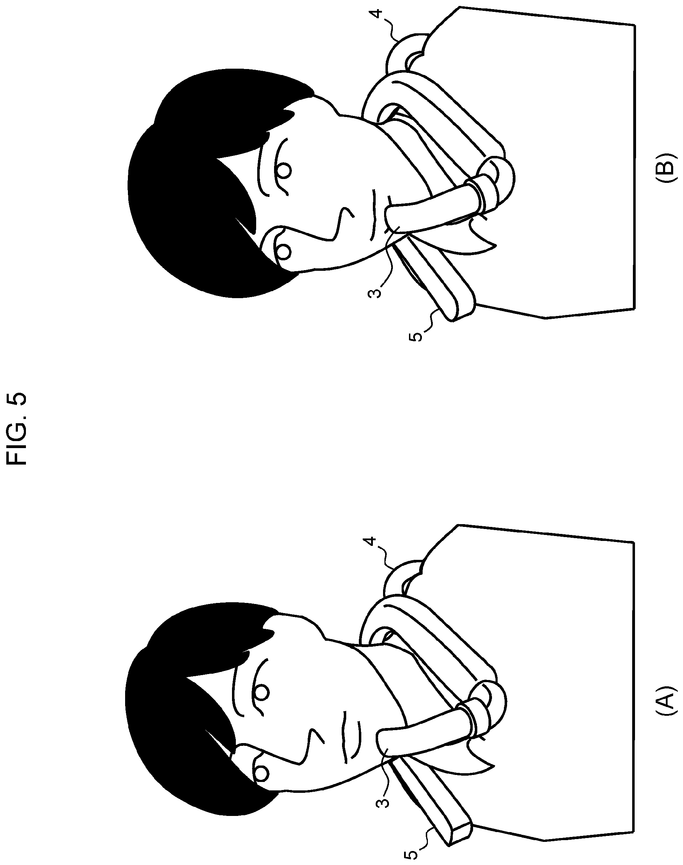

With the respiratory assistance system 1 according to the aforementioned configuration, the subject can align the mouthpiece 3 with vicinity of his or her lips by just rotating the neck holder 5 slightly with his or her hand while walking with the artificial respirator 2 with him or her via the shoulder belt 11 (Figure 5(A)).

Then, only by holding the mouthpiece 3 in his or her mouth as needed, the subject can immediately receive respiratory assistance by the artificial respirator 2 (Figure 5(B)). At that time, the subject can keep carrying the artificial respirator 2 and at the same time perform an operation necessary for respiratory assistance by using the operation interface 30 placed on the exposed surface of the artificial respirator 2 and can also easily replace the portable battery 32 provided on the exposed surface.

### (1-1) Experiment Result of Use of Respiratory Assistance System According to First Embodiment

Here, in the respiratory assistance system 1 as described above, an experiment was performed for checking whether respiratory assistance is performed in synchronization with timing of spontaneous respiration by a subject while walking or in a resting seated state and whether the gas pressure (12 to 25 [cmH2O]) set in consideration of the tidal volume of an adult is an effective artificial respirator pressure.

A 23-years old healthy male subject is registered as a subject of the experiment. The subject holds the mouthpiece 3 over his mouth and sits on a chair at a seated rest position. As a walking condition while walking, the subject reciprocates on a 5[m] straight line.

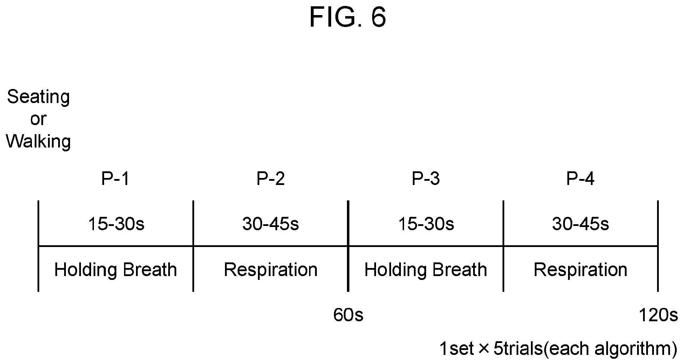

Figure 6 shows experiment steps (on a time axis from P-1 to P-4). First, in step P-1, the subject holds breathing for 15 to 30 seconds or a pleasant period of time (Holding Breath). Then, in step P-2, the subject performs light respiration properly until one minute passes from the start of the experiment (Respiration). After several natural respirations, the subject relaxes his respiratory muscle and receives respiratory assistance by the respiratory assistance system 1. The subject counts the number of respirations.

In step P-3, the subject holds breathing for 15 to 30 seconds or a comfortable period of time (Holding Breath). Then, in step P-4, the subject performs light respiration properly until two minutes pass from the start of the experiment (Respiration). After several spontaneous respirations, the subject relaxes his respiratory muscle and receives respiratory assistance by the respiratory assistance system 1. The subject counts the number of respirations.

Under the resting seated condition and the walking condition of the subject, the control unit 40 in the respiratory assistance system 1 attempts the detection algorithm for mouthpiece holding action five times for each of the conditions. It should be noted that the respiratory assistance ability such as the pressure, volume or the like of the gas output from artificial respirator 2 was evaluated based on the ventilation amount calculated from the flow rate value acquired from the flow rate sensor 42 within the blower apparatus 10. Furthermore, the ventilation amount was calculated by integrating in time the flow rate values. The integration start time and the integration end time are equal to the assistance start time and end time, respectively.

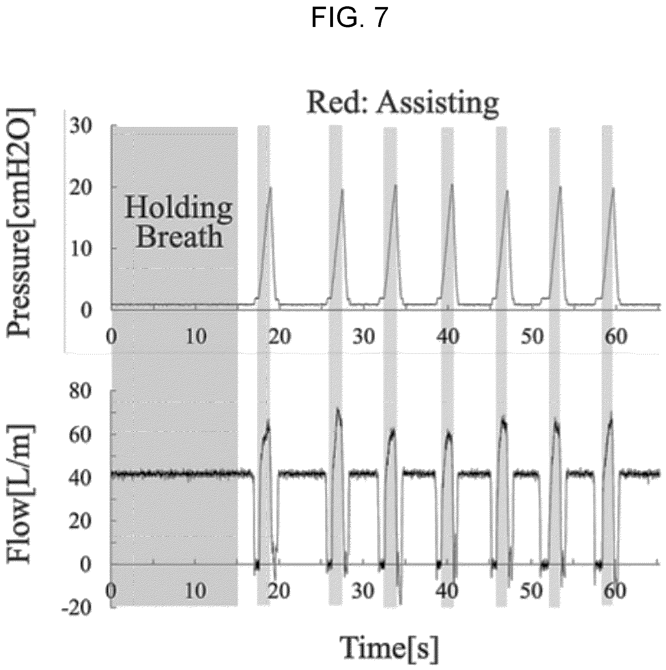

Figure 7 shows experiment results by the mouthpiece holding action detection algorithm. Referring to Figure 7, the left side shows breathing is held (Holding Breath) while the right side shows a state in which respiration is assisted by the respiratory assistance system 1 (Assisting). The flow rate (Flow) and pressure (Pressure) of the respiratory tract circuit did not change when the subject held breathing but changed when he was breathing.

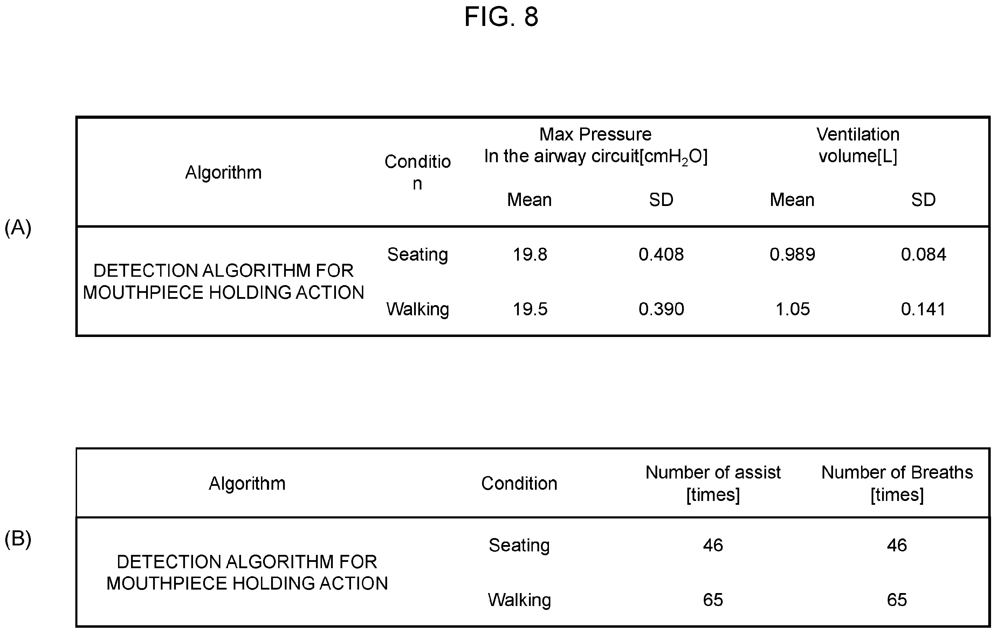

Figure 8(A) shows the number of respirations by the subject and the number of respiration assistances by the respiratory assistance system 1 according to the experiment. In a case where the detection algorithm for mouthpiece holding action was used, the number of respiration assistances by the respiratory assistance system 1 was matched with the number of respirations by the subject both in the rest-seated condition (Seating) and the walking condition (Walking).

Figure 8(B) shows an average value and a standard deviation of the maximum pressure within the respiratory tract circuit of the subject during the experiment in which respiratory assistance was performed. The average ventilation amount was calculated from the flow rate value. In a case where the detection algorithm for mouthpiece holding action was used, the average value of maximum pressures of the respiratory tract circuit in the rest-seated state (Seating) was about 20 [cmH2O] and the average value of the ventilation amount was about 1.0[L] while the average value of maximum pressures of the respiratory tract circuit in the walking state (Walking) was about 20 [cmH2O], and the average value of the ventilation amount was about 1.1[L].

According to this experiment result, it could be confirmed that the detection algorithm for mouthpiece holding actions was effective for spontaneous respiratory assistance in the rest-seated state and the walking state.

Furthermore, as a result of a pressure measurement experiment by the pressure sensor 43 within the blower apparatus 10, the maximum bearing pressure was approximately 20 [cmH2O]. Many of targets who need respiratory assistance can use a bearing pressure of 12 to 25 [cmH2O] safely and effectively, there is a possibility that actual targets can be assisted. Furthermore, as a result of a ventilation amount measurement experiment with the flow rate sensor 42 within the blower apparatus 10, since it was possible that a ventilation of about 1[L] within the laboratory and the amount of one ventilation of an adult is about 500[mL], it was recognized that a sufficient ventilation amount was acquired. Moreover, in a case where sufficient ventilation cannot be maintained with a pressure of 20 [cmH2O] such as a patient suffering an intractable neurological or muscle disease or a patient suffering a respiratory function disorder who has a low value of ventilation amount against unit pressure, the tidal volume of an adult patient can be controlled adaptively with the target pressure value of this system.

### (2) Configuration of Respiratory Assistance System According to Second Embodiment

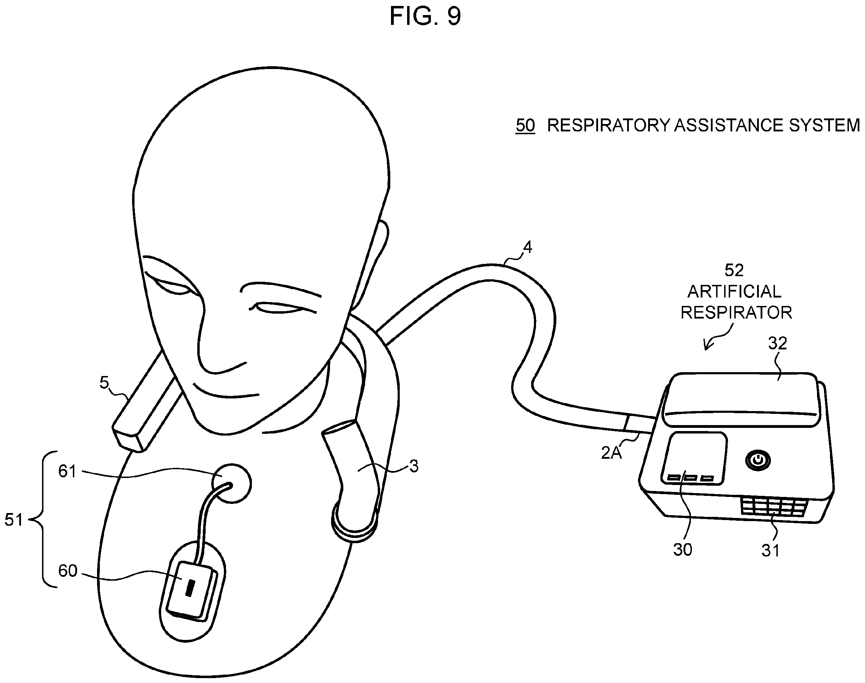

In a respiratory assistance system 50 shown in Figure 9 in which like references are given to the parts corresponding to Figure 1, a sensor unit 51 that is noninvasively and removably attached with reference to the chest of a subject is communicably connected in wireless manner, and it is configured such that detection data acquired from the sensor unit 51 is received by the artificial respirator 52.

This sensor unit 51 has a signal detecting unit 60 that detects a bioelectrical signal generated by shrinking and relaxing of the diaphragm with spontaneous respiration of the subject and a vibration detecting unit 61 that detects a lung activity with spontaneous respiration of the subject.

The signal detecting unit 60 has an electrode terminal group on a measurement surface of the apparatus body of the sensor unit 51 and detects a bioelectrical signal of a subject via the electrode terminal group. The vibration detecting unit 61 includes a sound collecting unit (not shown) connected to a connection terminal provided on a side surface of the apparatus body of the sensor unit 51 and collects, as an acoustic signal, sound of vibrations generated by a lung activity with spontaneous respiration of the subject. This acoustic signal also contains information on vibrations (non-audible region) with the heart or respiration for the subject that cannot be heard by the ears.

Note that an inner battery (such as lithium-ion battery, for example) for supplying sensor power to the signal detecting unit and the vibration detecting unit (sound collecting unit) is mounted in the sensor unit 51.

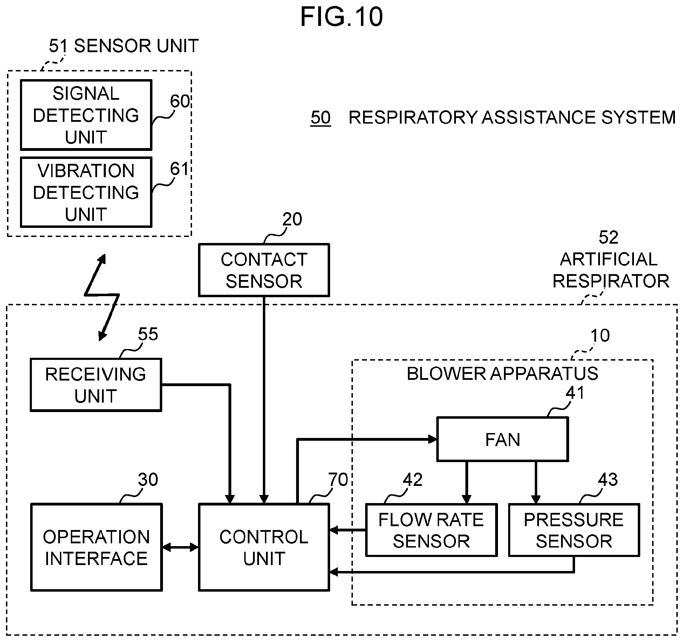

Actually, in the sensor unit 51, the measurement surface of the apparatus body in which the electrode terminal group of the signal detecting unit 60 is provided is positioned on the lower side of the chest of the subject, and the sound collecting unit as the vibration detecting unit 61 is attached near the chest of the subject. Additionally, the sensor unit 51 and the artificial respirator 52 are communicably connected in wireless manner by a short-range communication method, and it is configured such that detection data sent from the sensor unit 51 is received by a receiving unit 55 (see Figure 10 which will be described later) in the artificial respirator 52.

A control-related system of the artificial respirator 52 in the respiratory assistance system 50 is shown in Figure 10. In Figure 10 in which like references are given to parts corresponding to Figure 3, a control unit 70 in the artificial respirator 52 controls either one or both of the ventilation amount and air pressure of the pressurized air to be supplied to the respiratory circuit 4 for the blower apparatus 10 so as to be synchronized with inhalation or exhalation timing of the subject based on a detection result from the signal detecting unit 60, which is received by the receiving unit 55.

As a result, in the respiratory assistance system 50, when the subject holds the mouthpiece 3 in his or her mouth, the respiratory circuit 4 can be supplied with pressurized air in synchronization with abdominal breathing timing of the subject during respiratory assistance to the subject.

In the respiratory assistance system 50, a control unit 70 in the artificial respirator 52 controls either one or both of the ventilation amount and air pressure of the pressurized air to be supplied to the respiratory circuit 4 for the blower apparatus 10 so as to be synchronized with inhalation or exhalation timing of the subject based on a detection result from the vibration detecting unit 61, which is received by the receiving unit 55.

As a result, in the respiratory assistance system 50, when the subject holds the mouthpiece 3 in his or her mouth, the respiratory circuit 4 can be supplied with pressurized air in synchronization with timing of slight movements of the lung with spontaneous respiration of the subject during respiratory assistance to the subject.

Furthermore, while, in the respiratory assistance system 50, when the control unit 70 in the artificial respirator 52 controls the blower apparatus 10 to supply pressurized air to the respiratory circuit 4, the detection result from the signal detecting unit 60 and the detection result from the vibration detecting unit 61 are divided, the detection results from the signal detecting unit 60 and vibration detecting unit 61 may be combined.

In other words, the control unit 70 in the artificial respirator 52 controls either one or both of the ventilation amount and air pressure of the pressurized air to be supplied to the respiratory circuit 4 for the blower apparatus 10 so as to be synchronized with inhalation or exhalation timing of the subject based on a detection results from the signal detecting unit 60 and the vibration detecting unit 61.

As a result, in the respiratory assistance system 50, when the subject holds the mouthpiece 3 in his or her mouth, the respiratory circuit 4 can be supplied with pressurized air in synchronization with abdominal breathing timing of the subject during respiratory assistance to the subject and, at the same time, the artificial respirator 52 can allow the respiratory circuit 4 to be supplied with pressurized air to the artificial respirator 52 in synchronization with timing of slight movements of the lung with spontaneous respiration of the subject during respiratory assistance to the subject.

In this way, in the respiratory assistance system 50, when the subject performs abdominal breathing which moves the diaphragm upward and downward, synchronization is acquired with the abdominal breathing timing while, when the subject performs the costal breathing which does not move the diaphragm upward and downward, synchronization is acquired with breathing timing in accordance with slight movement of the lung with spontaneous respiration, so that the artificial respirator 52 allows the respiratory circuit to be supplied with pressurized air in an optimum state regardless of the breathing details of the subject.

Next, a spontaneous respiration detection algorithm using bioelectrical signals of a respiratory muscle by the control unit 70 is described. By utilizing a bioelectrical signal of the respiratory muscle which is measurable on the skin surface, there is a possibility that spontaneous respiration can be detected noninvasively and routinely. The bioelectrical signal is a minute signal which leaks to the body surface when a motor command from the brain is transmitted to the spiral cord, a motor neuron, and muscle fiber.

Although an involuntary movement is controlled by the medulla oblongata while a voluntary movement is controlled by the cerebral motor cortex, the respiratory muscle is constructed by a skeletal muscle (voluntary muscle) which can be moved voluntarily. For spontaneous respiration, since the external intercostal muscles and the diaphragm are muscles mainly used during inhalation, bioelectrical signals detected from those muscles can be detected.

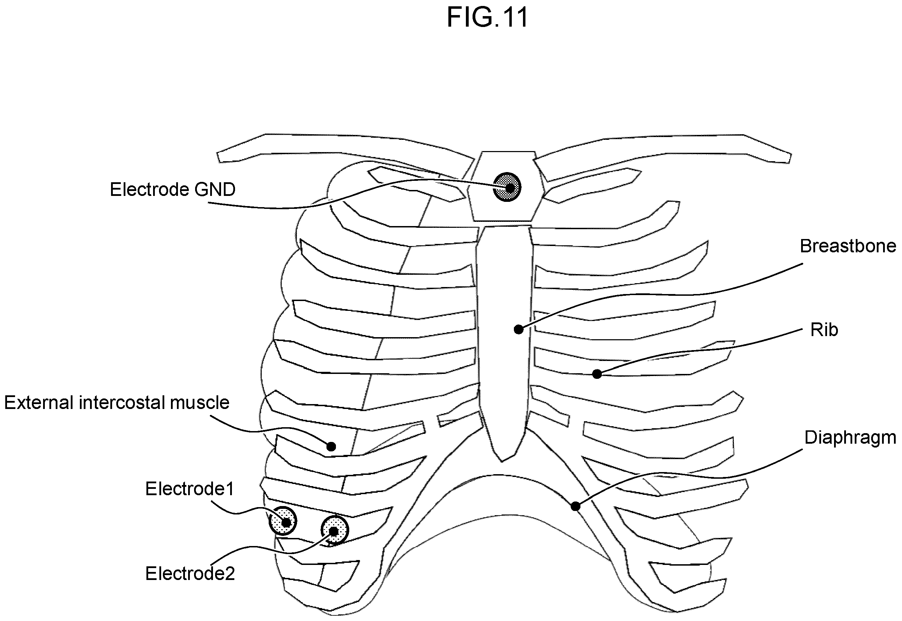

According to the present invention, as a result of an attempt of a measurement of a bioelectrical signal in the external intercostal muscle which is closer to the surface than the diaphragm, it was found that the bioelectrical signal from the external intercostal muscle is in a layer closer to the surface than the diaphragm. Figure 11 shows a positional relationship among breast bones (sternum, costal bone), the diaphragm, external intercostal muscles and positions of electrodes (electrode 1, electrode 2 and electrode GND) when measuring bioelectrical signals.

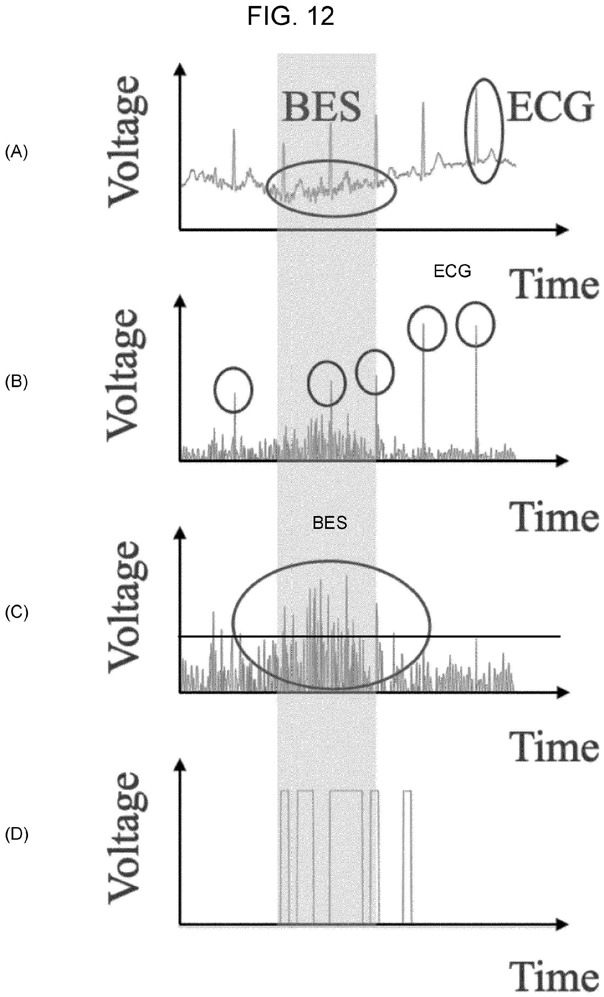

Here, with respect to the bioelectrical signal (BES), since the baseline of a measurement waveform for a motion artifact is not constant, instability of the baseline is removed. More specifically, a median filter is applied to a raw signal (raw data) of the bioelectrical signal shown in Figure 12(A), and the instability of the baseline is removed by subtracting a signal acquired by applying the median filter from the raw signal.

As shown in Figure 12(B), in order to extract a bioelectrical signal of the respiratory muscle, the control unit 70 removes spike noise due to an electrocardiogram (ECG) from the signal after the instability of the baseline is removed. The removal of the spike noise due to an electrocardiogram is performed by removing an outlier (abnormal value) by employing an average value x^(Equation (1) below) and a standard deviation σ(Equation (2) below) of the latest 13 spike noises xᵢ(I = 0 - 12).
[Equation 1] $\hat{x} = \frac{1}{13} {\prod }_{i = 0}^{12} {x}_{i}$
[Equation 2] $σ = \frac{1}{\sqrt{13}} {\sum }_{i = 0}^{12} \left|{x}_{i}-\hat{x}\right|$

From the calculation result of Equation (2), a conditional expression provided in Equation (3) below returns xₜ₋₁ if it is true or returns xt if it is false.
[Equation 3] ${x}_{t} \geq \hat{x} + 2 σ \left‖{x}_{t}<\hat{x}-2σ\right.$

In Equation (3), returning the previous value if it is false also plays a role of data imputation for avoiding sparsity of the signal.

Then, as shown in Figure 12(C), the control unit 70 counts the data amount exceeding a threshold for the bioelectrical signal toward an outlier removal signal, and, if the count exceeds a threshold (threshold for the number of times of exceeding), determines that the subject has performed spontaneous respiration and performs respiratory assistance.

According to the present invention, the thresholds are determined by try and error, and the threshold for the bioelectrical signal and the threshold for the number of times of exceeding are set as 500 [µV] and 5, respectively. When the respiratory assistance ends, the control unit 70 returns to a standby state (Figure 12(D)).

### (2-1) Experiment Result of Use of Respiratory Assistance System According to Second Embodiment

Here, in the respiratory assistance system 50 as described above, an experiment was performed for checking whether respiratory assistance is performed in synchronization with timing of spontaneous respiration by a subject while walking or in a resting seated state and whether the gas pressure (12 to 25 [cmH2O]) set in consideration of the tidal volume of an adult is an effective artificial respirator pressure.

This experiment was performed in a manner similar to the experiment steps shown in Figure 6, like the case of the first embodiment as described above. In the respiratory assistance system 50 according to a second embodiment, under the resting seated condition and the walking condition of the subject, the control unit 70 attempts the spontaneous respiration detection algorithm using a bioelectrical signal of a respiratory muscle as described above five times for each of the conditions.

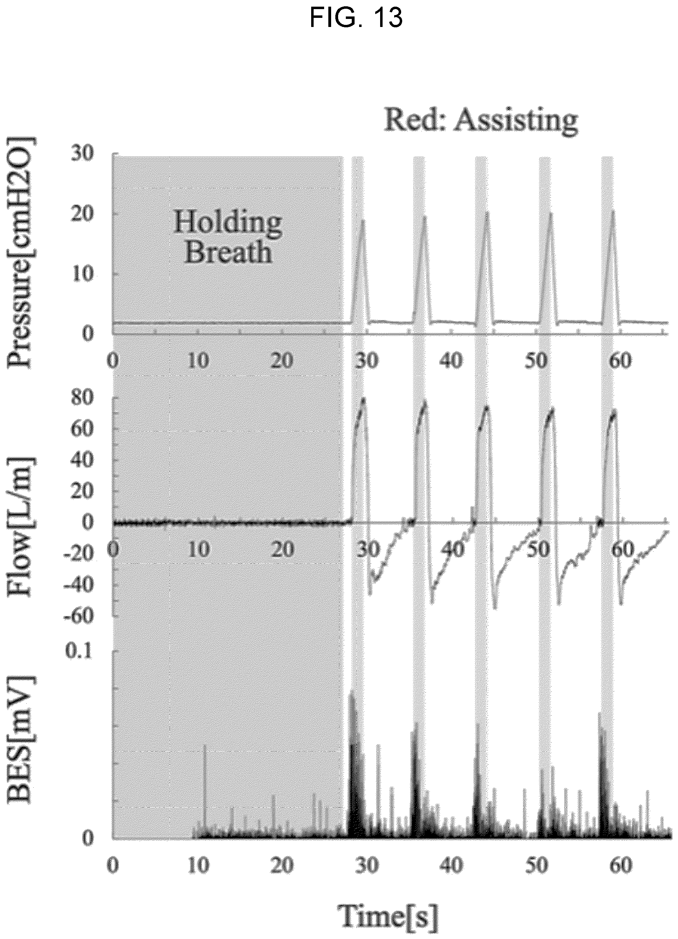

Figure 13 shows experiment results by the spontaneous respiration detection algorithm using bioelectrical signals of a respiratory muscle. Referring to Figure 13, the left side shows breathing is held (Holding Breath) while the right side shows a state in which respiration is assisted by the respiratory assistance system 1 (Assisting). The bioelectrical signal (BES) of a respiratory muscle did not change when the subject held breathing but changed when he was breathing.

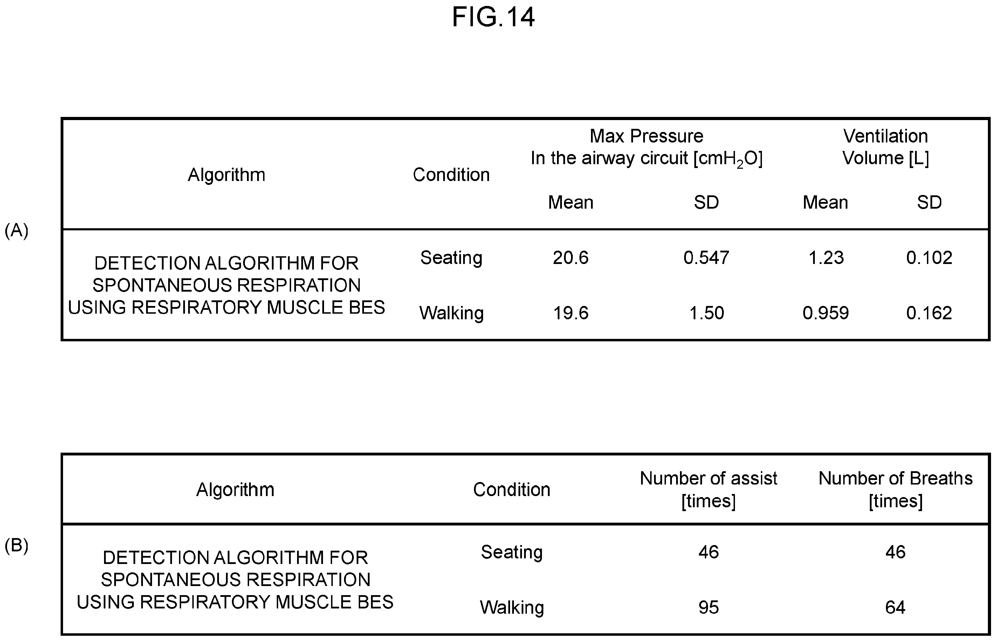

Figure 14(A) shows the number of respirations by the subject and the number of respiration assistances by the respiratory assistance system 50 according to the experiment. In a case where the spontaneous respiration detection algorithm using a bioelectrical signal of a respiratory muscle was used, the number of respiration assistances by the respiratory assistance system 50 was about 1.5 times of the number of respirations by the subject in both the rest-seated condition (Seating) and the walking condition (Walking).

Figure 14(B) shows an average value and a standard deviation of the maximum pressure within the respiratory tract circuit of the subject during the experiment in which respiratory assistance was performed. The average ventilation amount was calculated from the flow rate value. In a case where the spontaneous respiration detection algorithm employing a bioelectrical signal of a respiratory muscle was used, the average value of maximum pressures of the respiratory tract circuit in the rest-seated state (Seating) was about 20 [cmH2O] and the average value of the ventilation amount was about 1.2[L] while the average value of maximum pressures of the respiratory tract circuit in the walking state (Walking) was about 20 [cmH2O], and the average value of the ventilation amount was about 0.96 [L].

According to this experiment result, under a rest-seated condition, spontaneous respiration was detected by the spontaneous respiration detection algorithm using a bioelectrical signal of a respiratory muscle so that spontaneous respiratory assistance was performed. Conversely, under a walking condition, since the frequency of respiration assistances is higher compared to respiration of the subject, unintentional respiratory assistance was performed by the spontaneous respiration detection algorithm using a bioelectrical signal of a respiratory muscle.

Because of this, based on the intension and operation for respiration by the subject, another voluntary respiratory assistance algorithm for walking is combined so that voluntary respiratory assistance can be performed routinely.

When an intention to breath does not appear as a change in the flow rate such as a case with obstructive sleep apnea syndrome (SAS), the spontaneous respiration detection algorithm using a bioelectrical signal of a respiratory muscle detects respiration timing from the bioelectrical signal of the respiratory muscle so that respiratory assistance can be performed without preventing the respiration timing of the subject.

### (3) Other Embodiments

It should be noted that, having described, according to the first and second embodiments, the case where an electrostatic capacity switch is applied as the contact sensor 20 provided at an end part of the mouthpiece 3, the present invention is not limited thereto, and, if the contact state achieved when the subject holds the mouthpiece 3 in his or her mouth can be detected, a resistive membrane system switch, for example, may be applied which detects a press force to the circumference of the end part of the mouthpiece 3.

Furthermore, having described according to the first and second embodiments that the shoulder belt 11 is applied as a holder that holds the artificial respirator 2, 52 to the body, the present invention is not limited thereto, and the artificial respirator 2, 52 may be accommodated in a bag such as a pochette as the holder to be held on the body of the subject.

Furthermore, having described that, according to the first and second embodiments, a centrifugal micro blower is applied as the fan 41 within the blower apparatus 10, the present invention is not limited thereto, and a micro blower employing piezoelectric element (piezo actuator) may be applied. This piezoelectric micro blower amplifies at a C-MOS inverted from an oscillator to drive the piezoelectric element so as to play a role as an air pump. As a result, the size reduction and low noise can be significantly secured compared to a general centrifugal micro blower.

Moreover, having described that, according to the first and second embodiments, the flow rate sensor 42 and the pressure sensor 43 within the blower apparatus 10 are separate units, an air sensor module may be applied which can measure the pressure and flow rate of supplied air at all times.

Having described the case where, according to the second embodiment, the sensor unit 51 and the artificial respirator 52 are communicably connected in wireless manner, the present invention is not limited thereto, and the sensor unit 51 and the artificial respirator 52 may be connected in wired manner via, for example, a communication cable.

Although several embodiments have been described above, they are merely given for illustration purpose for explaining the present invention, and it is intended that the scope of the present invention is limited by the appended claims

### Reference Signs List

1, 50: respiratory assistance system, 2, 52: artificial respirator, 2A: intake port, 3: mouthpiece, 4: respiratory circuit, 5: neck holder, 10: blower apparatus, 11: shoulder belt, 20: contact sensor, 30: operation interface, 31: air filter, 32: portable battery, 40, 70: control unit, 41: fan, 42: flow rate sensor, 43: pressure sensor, 51: sensor unit, 55: receiving unit, 60: signal detecting unit, 61: vibration detecting unit

## Claims

1. A respiratory assistance system (1,50), comprising:
a portable artificial respirator (2,52) that noninvasively assists respiratory of a subject;
a respiratory circuit (4) having one end to which the artificial respirator is connected and another end to which a mouthpiece (3) is connected;
a neck holder (5) configured such that the mouthpiece is placed in vicinity of the mouth of the subject when the neck holder is attached to the neck of the subject; and
a contact sensor (20) that is provided at a tip of the mouthpiece and that detects a contact state when the subject holds the mouthpiece in his or her mouth,
wherein the artificial respirator controls a start or end of respiratory assistance to the subject based on a detection result of the contact sensor.

2. The respiratory assistance system according to claim 1, further comprising:
a signal detecting unit that is removably attached to a lower part of the chest of the subject and that detects a bioelectrical signal generated by shrinking and relaxing of the diaphragm with spontaneous respiration of the subject,
wherein the artificial respirator controls either one or both of the ventilation amount and air pressure of the pressurized air to be supplied to the respiratory circuit so as to be synchronized with inhalation or exhalation timing of the subject based on a detection result from the signal detecting unit.

3. The respiratory assistance system according to claim 1, further comprising:
a vibration detecting unit that is removably attached to a part near the chest of the subject and that detects a lung activity with spontaneous respiration of the subject,
wherein the artificial respirator controls either one or both of the ventilation amount and air pressure of the pressurized air to be supplied to the respiratory circuit so as to be synchronized with inhalation or exhalation timing of the subject based on a detection result from the vibration detecting unit.

4. The respiratory assistance system according to claim 1, further comprising:
a signal detecting unit that is removably attached to a lower part of the chest of the subject and that detects a bioelectrical signal generated by shrinking and relaxing of the diaphragm with spontaneous respiration of the subject; and
a vibration detecting unit that is removably attached to a part near the chest of the subject and that detects a lung activity with spontaneous respiration of the subject,
wherein the artificial respirator controls either one or both of the ventilation amount and air pressure of the pressurized air to be supplied to the respiratory circuit so as to be synchronized with inhalation or exhalation timing of the subject based on detection results from the signal detecting unit and the vibration detecting unit.

5. The respiratory assistance system according to any one of claims 1 to 4,
wherein the artificial respirator has a wearable size and weight so that the artificial respirator can be attached to the body of the subject and is held on the body of the subject via a holder.

6. The respiratory assistance system according to claim 5,
wherein the artificial respirator has a battery and an operation interface arranged in parallel on an exposed surface of the subject to which the artificial respirator is attached, the operation interface including a display function.

## Patentansprüche

1. Atemunterstützungssystem (1, 50), umfassend:
ein tragbares künstliches Beatmungsgerät (2, 52), das auf nichtinvasive Weise die Atemfunktion eines Individuums unterstützt;
einen Beatmungskreislauf (4) mit einem Ende, an dem das künstliche Beatmungsgerät angeschlossen ist, und einem anderen Ende, an dem ein Mundstück (3) angeschlossen ist;
eine Halshalterung (5), die derart ausgelegt ist, dass das Mundstück in der Nähe des Munds des Individuums platziert ist, wenn die Halshalterung an dem Hals des Individuums angebracht ist; und
einen Berührungssensor (20), der an einer Spitze des Mundstücks bereitgestellt ist und einen Berührungszustand, wenn das Individuum das Mundstück in seinem Mund hält, detektiert,
wobei das künstliche Beatmungsgerät einen Beginn oder ein Ende der Atemunterstützung für das Individuum basierend auf einem Detektionsergebnis des Berührungssensors steuert.

2. Atemunterstützungssystem nach Anspruch 1, ferner umfassend:
eine Signaldetektionseinheit, die entfernbar an einem unteren Teil des Brustkorbs des Individuums befestigt ist und ein bioelektrisches Signal, welches durch Zusammenziehen und Entspannen des Zwerchfells bei spontaner Atmung des Individuums erzeugt wird, detektiert,
wobei das künstliche Beatmungsgerät entweder eines oder beides aus der Lüftungsmenge und dem Luftdruck der unter Druck stehenden Luft, die dem Beatmungskreislauf zuzuführen ist, derart steuert, dass es mit einem Einatmungs- oder Ausatmungszeitpunkt des Individuums basierend auf einem Detektionsergebnis von der Signaldetektionseinheit synchronisiert ist.

3. Atemunterstützungssystem nach Anspruch 1, ferner umfassend:
eine Schwingungsdetektionseinheit, die entfernbar an einem Teil nahe dem Brustkorb des Individuums befestigt ist und eine Lungenaktivität bei spontaner Atmung des Individuums detektiert,
wobei das künstliche Beatmungsgerät entweder eines oder beides aus der Lüftungsmenge und dem Luftdruck der unter Druck stehenden Luft, die dem Beatmungskreislauf zuzuführen ist, derart steuert, dass es mit einem Einatmungs- oder Ausatmungszeitpunkt des Individuums basierend auf einem Detektionsergebnis von der Schwingungsdetektionseinheit synchronisiert ist.

4. Atemunterstützungssystem nach Anspruch 1, ferner umfassend:
eine Signaldetektionseinheit, die entfernbar an einem unteren Teil des Brustkorbs des Individuums befestigt ist und ein bioelektrisches Signal, welches durch Zusammenziehen und Entspannen des Zwerchfells bei spontaner Atmung des Individuums erzeugt wird, detektiert; und
eine Schwingungsdetektionseinheit, die entfernbar an einem Teil nahe dem Brustkorb des Individuums befestigt ist und eine Lungenaktivität bei spontaner Atmung des Individuums detektiert,
wobei das künstliche Beatmungsgerät entweder eines oder beides aus der Lüftungsmenge und dem Luftdruck der unter Druck stehenden Luft, die dem Beatmungskreislauf zuzuführen ist, derart steuert, dass es mit einem Einatmungs- oder Ausatmungszeitpunkt des Individuums basierend auf einem Detektionsergebnis von der Signaldetektionseinheit und der Schwingungsdetektionseinheit synchronisiert ist.

5. Atemunterstützungssystem nach einem der Ansprüche 1 bis 4,
wobei das künstliche Beatmungsgerät ein(e) tragbare(s) Größe und Gewicht aufweist, sodass das künstliche Beatmungsgerät an dem Körper des Individuums befestigt sein kann und über eine Halterung an dem Körper des Individuums gehalten wird.

6. Atemunterstützungssystem nach Anspruch 5,
wobei das künstliche Beatmungsgerät eine Batterie und eine Betriebsschnittstelle aufweist, die auf einer freiliegenden Fläche des Individuums, an der das künstliche Beatmungsgerät befestigt ist, parallel angeordnet ist, wobei die Betriebsschnittstelle eine Anzeigefunktion umfasst.

## Revendications

1. Système d'assistance respiratoire (1, 50), comprenant :
un respirateur artificiel portable (2,52) qui aide de manière non invasive un sujet à respirer ;
un circuit respiratoire (4) présentant une première extrémité à laquelle le respirateur artificiel est relié et une autre extrémité à laquelle un embout buccal (3) est relié ;
un support de cou (5) configuré de telle sorte que l'embout buccal soit placé à proximité de la bouche du sujet lorsque le support de cou est fixé au cou du sujet ; et
un capteur de contact (20) qui est prévu au niveau d'une pointe de l'embout buccal et qui détecte un état de contact lorsque le sujet tient l'embout buccal dans sa bouche,
dans lequel le respirateur artificiel commande un début ou une fin d'assistance respiratoire au sujet sur la base d'un résultat de détection du capteur de contact.

2. Système d'assistance respiratoire selon la revendication 1, comprenant en outre :
une unité de détection de signal qui est fixée de manière amovible à une partie inférieure de la poitrine du sujet et qui détecte un signal bioélectrique généré par le rétrécissement et le relâchement du diaphragme avec une respiration spontanée du sujet,
dans lequel le respirateur artificiel commande la quantité de ventilation et/ou la pression d'air de l'air comprimé à fournir au circuit respiratoire de manière à être synchronisé avec le cadencement d'inhalation ou d'exhalation du sujet sur la base d'un résultat de détection provenant de l'unité de détection de signal.

3. Système d'assistance respiratoire selon la revendication 1, comprenant en outre :
une unité de détection de vibration qui est fixée de manière amovible à une partie proche de la poitrine du sujet et qui détecte une activité pulmonaire avec une respiration spontanée du sujet,
dans lequel le respirateur artificiel commande la quantité de ventilation et/ou la pression d'air de l'air comprimé à fournir au circuit respiratoire de manière à être synchronisé avec le cadencement d'inhalation ou d'exhalation du sujet sur la base d'un résultat de détection provenant de l'unité de détection de vibration.

4. Système d'assistance respiratoire selon la revendication 1, comprenant en outre :
une unité de détection de signal qui est fixée de manière amovible à une partie inférieure de la poitrine du sujet et qui détecte un signal bioélectrique généré par le rétrécissement et le relâchement du diaphragme avec une respiration spontanée du sujet ; et
une unité de détection de vibration qui est fixée de manière amovible à une partie proche de la poitrine du sujet et qui détecte une activité pulmonaire avec une respiration spontanée du sujet,
dans lequel le respirateur artificiel commande la quantité de ventilation et/ou la pression d'air de l'air comprimé à fournir au circuit respiratoire de manière à être synchronisé avec le cadencement d'inhalation ou d'exhalation du sujet sur la base de résultats de détection provenant de l'unité de détection de signal et de l'unité de détection de vibration.

5. Système d'assistance respiratoire selon l'une quelconque des revendications 1 à 4,
dans lequel le respirateur artificiel présente une taille et un poids pouvant être portés de telle sorte que le respirateur artificiel puisse être fixé au corps du sujet et soit maintenu sur le corps du sujet via un support.

6. Système d'assistance respiratoire selon la revendication 5,
dans lequel le respirateur artificiel présente une batterie et une interface de fonctionnement agencées en parallèle sur une surface exposée du sujet à laquelle le respirateur artificiel est fixé, l'interface de fonctionnement comprenant une fonction d'affichage.
